# EUROPEAN PATENT SPECIFICATION

(11) **EP 1 328 277 B1**
(45) Date of publication and mention of the grant of the patent: **14.02.2007**
(21) Application number: 01980564.7
(22) Date of filing: 26.10.2001
(51) Int. Cl.: A61K 31/662, A61P 19/08

(54) **BISPHOSPHONIC COMPOUNDS FOR STRENGTHENING OF CORTICAL BONE**
BISPHOSPHONSÄUREDERIVATE ZUR STÄRKUNG VON KORTIKALKNOCHEN
DERIVES DE L'ACIDE BISPHOSPHONIQUE POUR RENFORCER L'OS CORTICAL

(30) Priority: 27.10.2000 FI 20002361; 27.10.2000 FI 20002362
(43) Date of publication of application: 23.07.2003
(73) Proprietor: Schering Aktiengesellschaft, 13353 Berlin (DE)
(72) Inventor: ÖSTERMAN, Thua, FIN-20320 TURKU (FI); HANNUNIEMI, Ritva, FIN-21620 KUUSISTO (FI); HENTUNEN, Teuvo, FIN-20740 TURKU (FI); LIUKKO-SIPI, Sirpa, FIN-23200 VINKKILÄ (FI); NIKANDER, Hannu, FIN-21330 PAATTINEN (FI); SELANDER, Katri, UAB Cancer Center, WTI Room T558, Birmingham, AL 35294-3300 (US); SELLMAN, Raija, FIN-20740 TURKU (FI); VÄÄNÄNEN, Kalervo, FIN-20880 TURKU (FI)
(74) Representative: Matilainen, Mirja Helena
(86) International application number: PCT/FI2001/000929
(87) International publication number: WO 2002/034269

(56) References cited:
- WO-A1-92/11267
- WO-A1-92/11269
- WO-A1-95/33466
- WO-A1-96/07418
- M. NISHIKAWA ET AL.: 'Biphosphonates act on osteoblastic cells and inhibit osteoclast formation in mouse marrow cultures' BONE vol. 18, no. 1, January 1996, pages 9 - 14, XP002908111
- FRASER P. COXON ET AL.: 'Protein geranylgeranylation is required for osteoclast formation, function and survival: inhibition by biphosphonates and GGTI-298' JOURNAL OF BONE AND MINERAL RESEARCH vol. 15, no. 8, 2000, pages 1467 - 1476, XP002908112
- C.W.G.M. LOWIK ET AL.: 'Migration and phenotypic transformation of osteoclast precursors into mature osteoclast: the effect of a biphosphonate' JOURNAL OF BONE AND MINERAL RESEARCH vol. 3, no. 2, 1988, pages 185 - 192, XP002908113
- D.E. HUGHES ET AL.: 'Inhibition of osteoclast-like cell formation by biphosphonates in long-term cultures of human bone marrow' J. CLIN. INVEST. vol. 83, June 1989, pages 1930 - 1935, XP002908114

## Description

### FIELD OF THE INVENTION

The present invention relates to the preparation of pharmaceutical compositions for strengthening cortical bone. Such a pharmaceutical composition can be used as such or in combination with other bone active treatments including bisphosphonates and specific estrogen receptor modifying agents (SERMs).

### BACKGROUND OF THE INVENTION

Bisphosphonates are used as therapeutic agents for the treatment of pathological bone destruction of various origin, such as osteolytic bone disease due to malignancy, Paget's disease, and osteoporosis. They are analogues of the physiologically occurring inorganic pyrophosphates. The basic P-C-P structure of the bisphosphonates makes it possible to design a great number of different compounds by changing the side chains of the carbon atom. A different approach is to form various derivatives of the phosphate groups.

In general, bisphosphonates inhibit osteoclast activity, that is of the cells which are responsible for bone resorption. Known bisphosphonates bound to the bone matrix, enter resorbing osteoclasts and reduce the activity of matured osteoclasts. They inhibit bone resorption both *in vitro* and *in vivo*. Limited absorption from the gastrointestinal tract, fast incorporation in bone tissue, and unchanged excretion in urine are all characteristics of the known bisphosphonates.

In the patents US 4,447,256, DE 28 31 578, JP 55089210, JP 55098105, JP 55043054, JP 55043055 processes are described for the preparation of some pyridinylaminomethylene bisphosphonic acid tetraalkylesters. The disclosed compounds are suggested to be used as herbicides.

US 5,866,556 on the other hand discloses pyridinylaminomethylene bisphosphonic acid tetraalkylesters with a high oral bioavailability and practically no affinity to bone. According to this patent, the disclosed compounds are said to have less side effects without a loss of their antiresorptive activity. Also EP patents 563107 and 563096 disclose substituted methylene bisphosphonic acid in partial ester form for the treatment of various bone diseases, such as for the prevention of bone resorption and osteoporosis. WO 9 607 418 discloses parathyroid hormone (PTH) combinations, including PTH bisphosphonate combinations which may increase bone mass.

It has now been discovered that a group of methylene bisphosphonic acid esters exhibits an inhibitory or suppressing effect on osteoclast formation, that is they affect osteoclast formation at an early stage of this osteoclastogenesis process but do not have a direct effect on the bone.

It has been found that it is possible to suppress the osteoclastic activity at an early stage of the osteoclast formation process, rather than to exert an effect on the mature osteoclast. This is clearly evidenced as a strengthening effect of specifically the cortical bone. An effect is also seen in the density of the trabecular bone, but as far as the overall strengthening of the bone is concerned, a strengthening of the denser cortical bone is more important than a strengthening of the trabecular bone. The invention is especially useful for the preparation of pharmaceutical compositions for treatment to combat weakening of cortical bone, in addition to trabecular bone, in a postmenopausal female, a child, an elderly male, in a patient treated with corticosteroids, cancer chemotherapies or other bone strength weakening therapies, in a patient with cancer metastases in the bone, and in a cancer patient whose bone strength is weakened by local or systemic factors (cytokines *etc*.) secreted by the cancer cells.

The finding that has been made in the course of the invention can be applied in the use for strengthening or combatting weakening of cortical bone.

It has also been established that the active agent used does not bind to bone, as a consequence of which it is not retained in the organism, which fact is important from the point of view of side-effects. Further, the compounds used do not inhibit remodelation of the bone, *i*.*e*. the normal regeneration of the bone wherein part of the bone is resorbed and new bone is formed. This is especially important in the treatment of children and fertile women.

Known bisphosphonates bind to the bone and are retained there even for long periods of time. They also inhibit all bone resorptions, including the normal remodelation of the bone. Consequently, they cannot be used for example in the treatment of children and fertile women.

The present invention is directed to the use of compounds of the formula I' 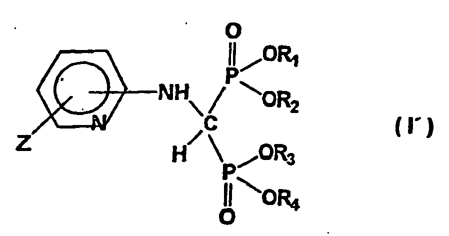 wherein Z has the meaning of hydroxy or protected hydroxy which is a lower alkoxy group with 1 to 4 carbon atoms, an aryloxy group, such as a benzyloxy group, or an acyloxy group, such as a lower alkyl acyloxy group, for example an acetoxy group, a pivaloyloxy-, aryl- or alkylsilyloxy group, and the groups R₁ to R₄ are the same or different, and mean an alkyl group with 1- 5 carbon atoms, whereby one of the groups R₁ to R₄ can also mean hydrogen, for the preparation of a pharmaceutical composition for strengthening cortical bone or combating weakening of the cortical bone.

Z in the above mentioned formula is preferably hydroxy, and the groups R₁ to R₄ are the same, and are preferably ethyl groups. Such a compound is especially [[(3-hydroxy-2 pyridinyl)amino]methylidene]bisphosphonic acid tetraethylester.

The said compounds as well as their manufacture is as such known, see for example US 5,866,556, EP 563107 and 563096.

The pharmaceutical compositions made according to the invention can be administered for example orally, parenterally, topically or rectally by means of any pharmaceutical formulation useful for said administration, and containing the active agent in a therapeutically effective and acceptable amount together with pharmaceutically acceptable carriers, adjuvants or vehicles known in the art. The manufacture of such pharmaceutical formulations is well known in the art.

Thus the pharmaceutical composition may be in a dosage form suitable for oral use, such as tablets, capsules, liquid dosage forms, such as solutions, suspensions, emul-sions, syrups, gels. All such formulations are made using *per se* known formulation techniques as well as known carriers, adjuvants and additives. Suitable excipients for oral administration forms are fillers, those for example for use in tablets including sugars, such as lactose, mannitol, sucrose, cellulose preparations, such as starch, gelatins, methyl cellulose, hydroxypropyl cellulose, microcrystalline cellulose, or silicified microcrystalline cellulose.

The compounds of formula I' may also be administered parenterally, for example as infusions or injections, using aqueous or oily suspensions, emulsions, or dispersions containing the active agent in combination with conventional pharmaceutically acceptable excipients, such as buffers, suspension and/or dispersion agents, preservatives. Also contempleted are formulations for topical or transdermal use in the form of creams, ointments, jellies, solutions, suspensions , or in the form of transdermal delivery systems, all of which are known to the person skilled in the art. For rectal use, the compounds are combined with a substance suitable for rectal administration such as waxes or fats or other known suppository bases. It is also possible to administer the compounds in the form of slow release formulations, the various forms of which are well known in the art. Another mode of administration is in the form of implants, such as subcutaneous implants, or as prosthetic implants, such as by treating the prosthesis with the compound to be administered, or in the form of bone cements.

The therapeutic dose to be given to a patient in need of treatment will vary depending *i*.*a*. on the body weight and age of the patient, the particular condition being treated, as well as the manner of administration and are easily determined by a person skilled in the art. A suitable amount can thus vary within wide limits, and can easily be established by a skilled physician. For most purposes an oral dose of appr. 0.01 to 15 mg/kg body weight/day would be suitable, for example when given 1 to 4 times daily. Such a dose means a daily dose of appr. 1 to 1200 mg for a subject weighing 80 kg.

### TESTING OF THE ACTIVITY ON CELL CULTURES (REFERENCE EXAMPLES)

The inhibitory activity of a representative compound to be used according to the invention, namely [[(3-hydroxy-2-pyridinyl)amino]methylidene]bisphosphonic acid tetraethylester (test compound, TC), was tested on different rat and mouse cell cultures, in order to assess its activity as an osteoclast formation inhibitor.

### Cultures used

### A. Mouse bone marrow culture

This culture system is the most used method to study osteoclast differentiation (Takahashi *et al*. 1988a). Mouse marrow cells can be induced to form active osteoclasts in 6-8 days in the presence of an osteotropic factor, such as 1,25(OH)₂D₃, parathyroid hormone, interleukin-1 or prostaglandin E₂ (Takahashi *et al*. 1988b, Shinar *et al.* 1990, Collins and Chambers 1992, Jimi *et al*. 1999). Typically, 10⁶ cells are cultured/ml of media for 6 days. Then, the cells are fixed with 3 % paraformaldehyde in PBS and stained for osteoclast marker, such as tartrate-resistant acid phosphatase (TRAP). Sigma's immunohistochemical kit can be used. Multinucleated, TRAP-positive cells containing at least 3 nuclei are counted as osteoclasts (Hentunen *et al*. 1998).

### B. Mouse spleen cell culture

Spleen has been shown to contain a population of early osteoclast precursors that can be induced to differentiate into osteoclasts in the presence of stromal cells, which have the capacity to support osteoclast formation (Udagawa *et al*. 1989). Without these supporting stromal cells, spleen cells do not differentiate into osteoclasts.

### C. Mouse osteoblast culture

Bone marrow stromal cells have long been known as a source of osteoprogenitor cells (Owen and Friedenstein 1988). Mouse bone marrow stromal cells are commonly induced to form preosteoblasts by culturing them for 6 days in the presence of glucocorticoid, ascorbic acid and sodium β-glycerophosphate. These preosteoblasts can be further induced to form active osteoblasts by continuing the cultures for two more weeks in the presence of estrogen (17β- estradiol). During this culture period cells begin to express increasing levels of osteoblast markers, such as alkaline phosphatase, type I collagen and osteocalcin, and finally they are able to form bone. Bone formation is detected by bone nodule formation and calcium deposition in the cultures (Qu *et al*. 1998).

### D. Mouse spleen cell/osteoblast co-culture

Spleen has been shown to contain a population of early osteoclast precursors that can be induced to differentiate into osteoclasts in the presence of stromal cells, which have the capacity to support osteoclast formation (Udagawa *et al*. 1989). Without these supporting stromal cells, spleen cells do not differentiate into osteoclasts. A co-culture system was developed, which contains mouse spleen cells and primary mouse osteoprogenitor cells. Osteoprogenitor cells were prepared from mouse marrow as described in section C.

### E. Rat osteoclast culture

Osteoclasts can be isolated from long bones of several species and they are cultured on bone or dentine slices to study their resorption activity (Boyde *et al*. 1984, Chambers *et al*. 1984). Mostly, either rat or rabbit osteoclasts are used. They are isolated from new-born animals and cultured for 1-2 days. The resorption activity is determined either by counting the number of resorption pits on bone/dentine slices or the total resorption area is measured using a phase contrast microscope and image analysis system. We are routinely using isolated rat osteoclasts and we culture them on bovine bone slices for two days, after which we fix the cells. Cells are stained with toluidine blue and counted under the microscope (Arnett and Dempster 1987, Lakkakorpi *et al*. 1989). Then the cells are removed by brushing them and the resorption pits are stained with WGA lectin, which binds to resorption lacuna in the bone slice. The number and area of pits are quantified (Selander *et al*. 1994).

The concentration range of the test compound in these assays was 10⁻¹⁰ - 10⁻⁵ M.

### Results of the culture studies

### A. Mouse bone marrow culture

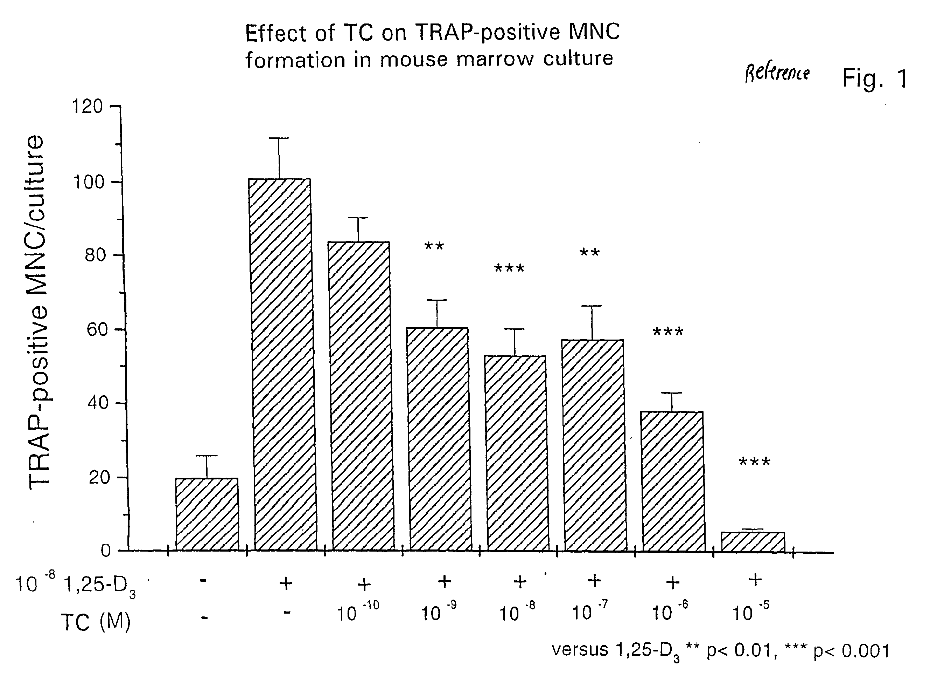

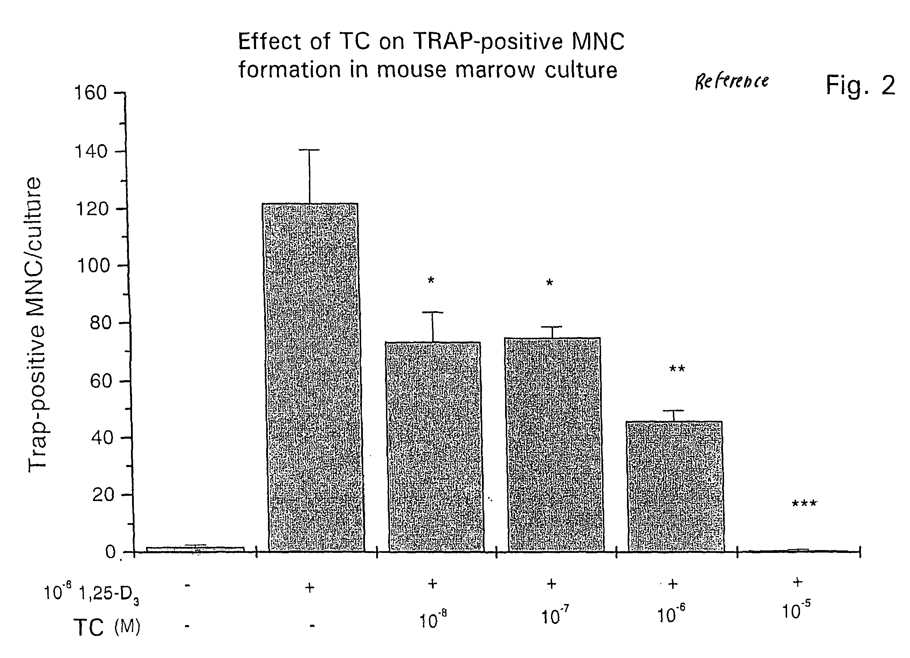

The test compound dose dependently inhibited both 1,25(OH)₂D₃- and PTH- induced osteoclast formation in bone marrow culture. 60% inhibition in osteoclast formation was obtained at 10⁻⁶ M. The test compound at 10⁻⁵ M was found to inhibit completely TRAP-positive MNC formation without having toxic effect on other cells (Fig. 1 & Fig. 2).

### B. Mouse spleen cell culture

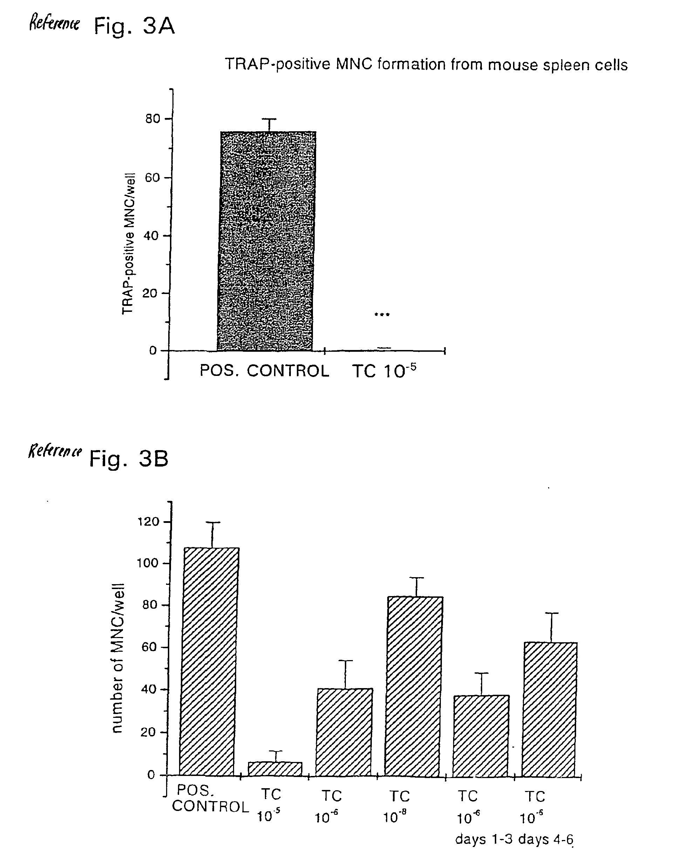

The test compound also dose dependently inhibited osteoclast formation in spleen cell culture. 60% inhibition was obtained at 10⁻⁶ M concentration as in the marrow culture system. At 10⁻⁵ M it completely blocked the TRAP-positive MNC formation (Fig 3). When the compound was present only at days 1-3, the inhibition of osteoclast formation was more than 60%, whereas when it was present on days 4-6, the inhibition was approximately 40 % (Fig 3). This suggests that the said compound is affecting the early steps of osteoclast maturation. Because no osteoblastic or stromal cells were present in the culture, it appears that the compound affects directly osteoclast precursors. The mechanism of action, however, still remains to be solved. However, at 10⁻⁵ M it totally blocked osteoclast-like cell formation and no TRAP-positive MNC or mononuclear cells were present in the culture (data not shown). This suggests that the compound inhibited osteoclast precursor cell differentiation before it started to express TRAP.

### C. Mouse spleen cell/osteoblast coculture

When these two cell types were cultured in the presence of 1,25(OH)₂D₃, several osteoclasts were formed. Spleen cells alone with 1,25(OH)₂D₃ were not able to differentiate into osteoclasts. Mouse osteoprogenitor cells, which were able to support osteoclast formation, were incubated first with 10⁻⁵ M of the said test compound for 24 h and then these cells were used in the co-culture with mouse spleen cells. It was found that so-treated preosteoblasts supported osteoclast formation in the similar manner as non-treated preosteoblasts (data not shown). This further supports the idea, that the test compound has direct inhibitory effect on osteoclast precursors.

### D. Rat osteoclast culture

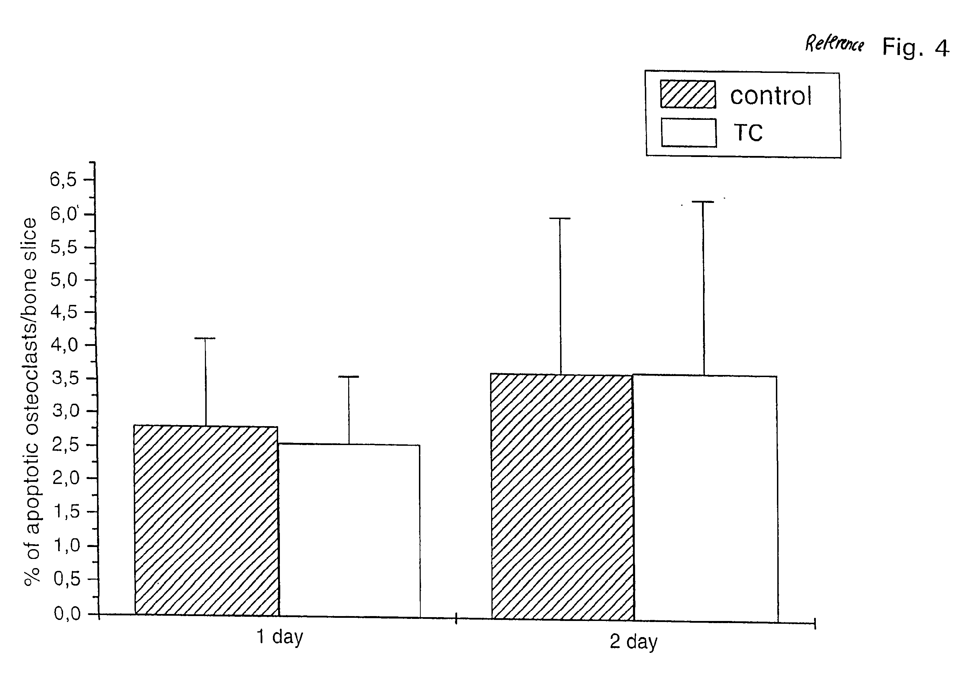

The compound did not have any effect on osteoclast-mediated bone resorption (data not shown). It did not have any effect on osteoclast apoptosis, when osteoclasts were cultured for 1-2 days on bovine bone slices (Fig. 4).

### TESTS ON OVARIECTOMIZED RATS

The activity of [[(3-hydroxy-2-pyridyl)amino]methylidene]bisphosphonic acid tetraethylester (TC) has been tested in ovariectomized rats. In the said tests, the compound to be tested was administered to six-month old rats. The control rats were sham-operated or overiectomized and given vehicle. The compound was given to ovariectomized rats orally 7 days a week for 12 weeks beginning immediately after the surgery. The doses were 10, 50 and 100 mg/kg/day. At the end of the study, the mineral density and the geometry of the bone was measured with pQCT (peripheral quantitative computed tomography). The strength of the bone was measured with electromechanical materials testing machine. The sites of measurement were the proximal end of the tibia (not strength), the lumbar vertebra, the femoral neck and the tibial mid-diaphysis. The parameters to be measured were the bone density of the trabecular or cortical bone, the cortical area and strength of the bone.

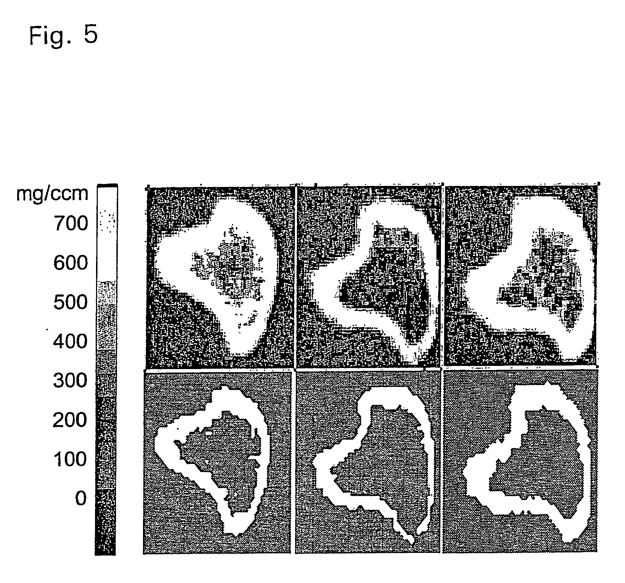

In the Fig. 5 the pQCT images are shown. In the upper row, from left to right, there is shown the bone mineral densities of a cross-section of the proximal tibia of a normal (sham-operated) rat, of an ovariectomized rat, and of an ovariectomized rat treated with the compound to be tested. From the results it is evident that the bone density in an ovariectomized rat is significantly reduced as compared to a normal rat, and also that this change is effectively inhibited by the compound tested. The lower row shows the corresponding measuring results for the cortical bone for a normal rat, an ovariectomized rat, and for an ovariectomized rat treated with the compound to be tested (counted from left to right). As can be seen, ovariectomy clearly reduces the thickness of the cortical bone as compared to the normal situation, which change can be inhibited with the compound tested.

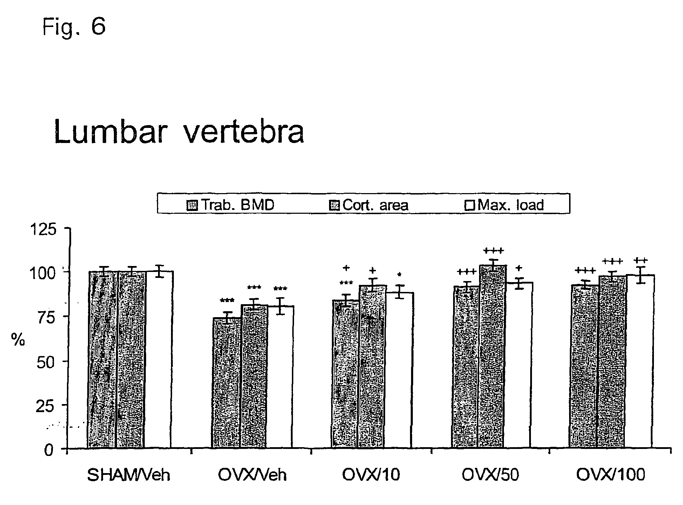

Fig. 6 shows that ovariectomy reduces the density of the trabecular bone and the area and strength of the cortical bone in the lumbar vertebra. The two higher doses of the tested compound, namely 50 and 100 mg/kg/day, inhibit these changes.

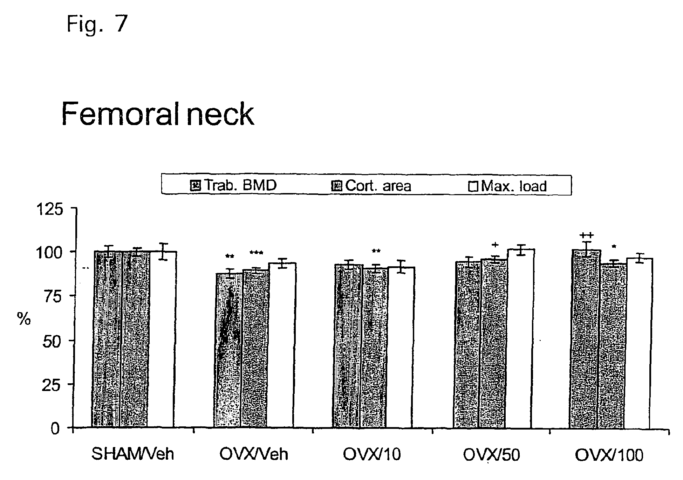

Fig. 7 in turn shows that 50 mg/kg/day of the compound tested inhibited the change in the cortical area in the femoral neck caused by ovariectomy; a dose of 100 mg/kg/day of the compound tested inhibited the change in the trabecular density.

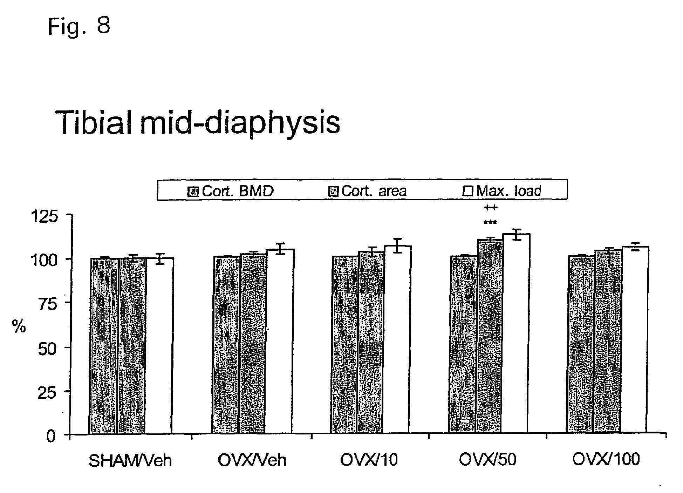

In the tibial mid-diaphysis, ovariectomy did not cause substantial changes, however, a dose of 50 mg/kg/day results in an increase of the cortical area as compared to a normal rat and an ovariectomized rat. Also the strength improved, although not to a significantly meaningful degree (Fig. 8).

The above results show that the beneficial effect on cortical bone is obtained in accordance with the invention, and that the compounds affect the osteoclast formation process rather than mature osteoclasts.

### TESTS IN A METASTASIS MODEL IN MOUSE (REFERENCE EXAMPLES)

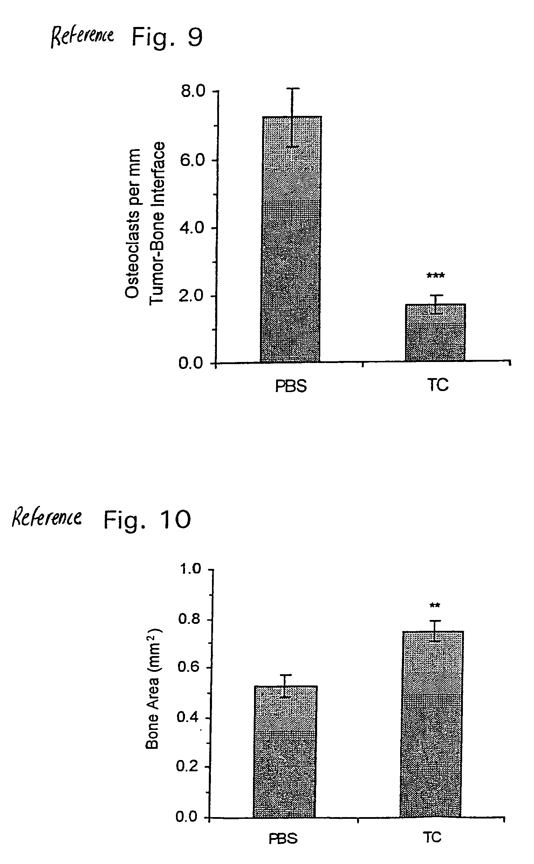

The effect of the test compound has been tested in a metastasis model in the mouse. 14 days after inoculation with human breast cancer cells, when the radiologically detected osteolytic bone lesions were found, the animals were treated either with PBS (control) or the test compound (50 mg/kg/day, s.c.) for 10 days. At the end of the study, osteoclast number and bone area were measured histomorphometrically. The test compound decreased significantly the number of osteoclasts at the tumor-bone interface as compared to the control (Fig. 9). Treatment with the test compound inhibited also tumor induced decrease in bone area at the site of metastases (Fig. 10).

### EXAMPLE 1

A pharmaceutical composition for oral use in the form of a tablet can be prepared using the following ingredients (per tablet)

| | |
|---|---|
| [[(3-hydroxy-2-pyridinyl)amino]methylene]bisphosphonic acid tetraethyl ester | 40.0 mg |
| Microcrystalline cellulose | 20.0 mg |
| Lactose | 67.0 mg |
| Starch | 10.0 mg |
| Talc | 4.0 mg |
| Magnesium stearate | 1.0 mg |

The ingredients are compounded into a tablet using conventional tablet making methods. If desired, the tablet may be coated with an enteric coating to control the point of release of the active agent.

### EXAMPLE 2

A pharmaceutical composition in the form of a capsule can be prepared using the following ingredients (per capsule)

| | |
|---|---|
| [[(3-hydroxy-2-pyridinyl)amino]methylene]bisphosphonic acid tetraethyl ester | 10.0 mg |
| Starch | 20.0 mg |
| Magnesium stearate | 1.0 mg |

The ingredients are combined in a known manner to form a mixture to be filled in a capsule of suitable size.

### REFERENCES

Arnett TR, Dempster DW (1987) A comparative study of disaggregated chick and rat osteoclasts in vitro: Effects of calcitonin and prostaglandins. Endocrinology 120: 602-608.

Boyde A, All NN, Jones SJ (1984) Resorption of dentine by isolated osteoclasts *in vitro.* Br Dent J 156: 216-220.

Chambers TJ, Revell PA, Fuller K, Athanasou NA (1984) Resorption of bone by isolated rabbit osteoclasts. J Cell Sci 66: 383-399.

Collins DA, Chambers TJ (1992) Prostaglandin E2 promotes osteoclast formation in murine hematopoietic cultures through an action on hematopoietic cells. J Bone Miner Res 7: 555-561.

Hentunen TA, Reddy SV, Boyce BF, Devlin R, Park H-R, Chung H, Selander K S, Dallas M, Kurihara N, Galson DL, Goldring SR, Koop, BA Windle JJ, Roodman GD (1998) Immortalization of osteoclast precursors by targeting bcl-XL and simian virus 40 large T antigen to the osteoclast lineage in transgenic mice. J Clin Invest 102: 88-97.

Jimi E, Nakamura I, Duong LT, Ikebe T, Takahashi N, Rodan GA, Suda T (1999) Interleukinl induces multinucleation and bone-resorting activity of osteoclasts in the absence of osteoblasts/stromal cells. Exp Cell Res 247: 84-93.

Lakkakorpi P, Tuukkanen J, Hentunen T, Järvelin K, Väänänen HK (1989) Organization of osteoclast microfilaments during the attachment to bone surface in vitro. J Bone Miner Res 4: 817-825.

Owen M, Friendenstem AJ (1988) Stromal stem cells: Marrow-derived osteogenic precursors. Ciba Found Symp 136:42-60.

Qu Q, Perälä-Heape M, Kapanen A, Dahllund J, Salo J, Väänänen, HK, Härkönen, P (1988) Estrogen enhances differentiation of osteoblasts in mouse bone marrow culture. Bone 22:201-209.

Selander K, Lehenkari P, Väänänen HK (1994) The effects of bisphosphonates on the resorption cycle of isolated osteoclasts. Calcif Tissue Int 55: 368-375.

Shinar DM, Sato M, Rodan GA (1990) The effect of hemopoietic growth factors on the generation of osteoclast-like cells in mouse bone marrow cultures. Endocrinology 126: 1728-1735.

Takahashi N, Yamana H, Yoshiki S, Roodman GD, Mundy GR, Jones SJ, Boyde A, Suda T (1988a) Osteoclast-like cell formation and its regulation by osteotropic hormones in mouse bone marrow cultures. Endocrinology 122:1373-1382.

Takahashi N, Akatsu T, Sasaki T, Nicholson GC, Moseley JM, Martin TJ, Suda T (1988b) Induction of calcitonin receptors by 1α,25-dihydroxyvitamin D3 in osteoclast-like multinucleated cells formed from mouse bone marrow cells. Endocrinology 123: 1504-1510.

Udagawa N, Takahashi N, Akatsu T, Sasaki T, Yamaguchi A, Kodama H, Martin TJ, Suda T (1989) The bone marrow-derived stromal cell lines MC3T3-GA/PA6 and ST2 support osteoclast-like cell differentiation in cocultures with mouse spleen cells. Endocrinology 125: 1805-1813.

## Claims

1. Use of compounds of the formula I' 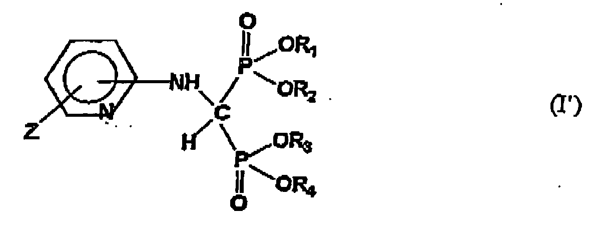 in which formula
Z has the meaning of hydroxy, or protected hydroxy which is a C₁ to C₄ alkoxy, aryloxy, acyloxy, aryl- or alkylsilyloxy group, and the groups R₁ to R₄ are the same or different, and mean an alkyl group with 1 - 5 carbon atoms, whereby one of the groups R₁ to R₄ can also mean hydrogen, for the preparation of a pharmaceutical composition for treatment to strengthen cortical bone or combating weakening of cortical bone.

2. The use according to claim 1 wherein Z is hydroxy.

3. The use according to claim 1, **characterized in that** the compound of formula I' is [[(3-hydroxy -2-pyridinyl)amino]methylidene]bisphosphonic acid tetraethylester.

## Patentansprüche

1. Verwendung von Verbindungen der Formel I' 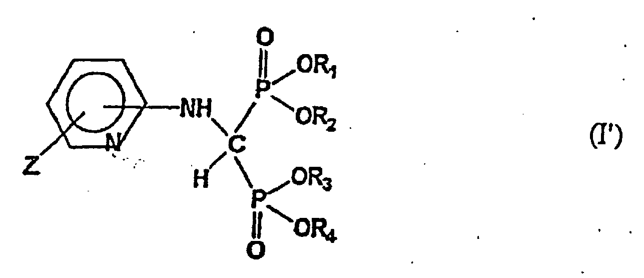 wobei in dieser Formel
Z Hydroxy oder geschütztes Hydroxy, welches ein C₁-C₄-Alkoxy-, Aryloxy-, Acyloxy-, Aryl- oder Alkylsilyloxyrest ist, bedeutet, und die Reste R₁ bis R₄ gleich oder verschieden sind und einen Alkylrest mit 1 bis 5 Kohlenstoffatomen bedeuten, wobei einer der Reste R₁ bis R₄ auch Wasserstoff bedeuten kann, für die Herstellung eines Arzneimittels zur Behandlung zur Stärkung der Kortikalis oder zur Bekämpfung der Schwächung der Kortikalis.

2. Verwendung gemäß Anspruch 1, wobei Z Hydroxy ist.

3. Verwendung gemäß Anspruch 1, **dadurch gekennzeichnet, dass** die Verbindung der Formel I' [[(3-Hydroxy-2-pyridinyl)amino]methyliden]bisphosphonsäuretetraethylester ist.

## Revendications

1. Utilisation de composés de formule I' 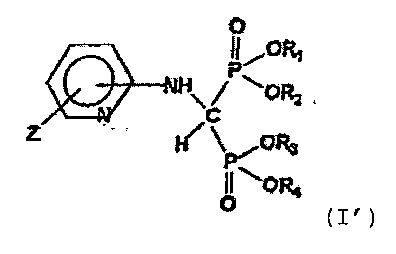 dans laquelle
Z est un hydroxy, ou un hydroxy protégé qui est un groupe alcoxy, aryloxy, acyloxy, aryl- ou alkylsilyloxy en C₁ à C₄, et les groupes R₁ à R₄ sont identiques ou différents, et sont un groupe alkyle comportant de 1 à 5 atomes de carbone, moyennant quoi l'un des groupes R₁ à R₄ peut aussi être un hydrogène, dans la préparation d'une composition pharmaceutique utilisée dans un traitement destiné à renforcer l'os cortical ou à combattre la fragilisation de l'os cortical.

2. Utilisation selon la revendication 1 dans laquelle Z est un hydroxy.

3. Utilisation selon la revendication 1, **caractérisée en ce que** le composé de formule I' est l'ester tétraéthylique de l'acide [[(3-hydroxy-2-pyridinyl)amino]méthylidène]diphosphonique.
